# EUROPEAN PATENT SPECIFICATION

(11) **EP 3 384 001 B1**
(45) Date of publication and mention of the grant of the patent: **25.12.2019**
(21) Application number: 16794211.9
(22) Date of filing: 09.11.2016
(51) Int. Cl.: C12N 1/14, C12N 1/16, C12N 1/20, C12Q 1/04, C12Q 1/22

(54) **CHEMICALLY DEFINED MEDIA FOR THE DETECTION OF MICROORGANISMS**
CHEMISCH DEFINIERTE MEDIEN ZUM NACHWEIS VON MIKROORGANISMEN
MILIEUX CHIMIQUEMENT DÉFINIS PERMETTANT LA DÉTECTION DE MICRO-ORGANISMES

(30) Priority: 03.12.2015 EP 15197739
(43) Date of publication of application: 10.10.2018
(73) Proprietor: Merck Patent GmbH, 64293 Darmstadt (DE)
(72) Inventor: RAYNER, Michael, Howard, 64342 Seeheim-Jugenheim (DE); KELLY, Sven, 64342 Seeheim-Jugenheim (DE); ANTHES, Bettina, 64711 Erbach (DE); SONNTAG, Ute, 64546 Moerfelden Walldorf (DE); HEDDERICH, Reiner, 69493 Hirschberg (DE)
(86) International application number: PCT/EP2016/001848
(87) International publication number: WO 2017/092842

(56) References cited:
- WO-A1-2009/026635
- WO-A1-2014/019696
- WO-A2-01/44278
- RIJN VAN DE I. ET AL: "GROWTH CHARACTERISTICS OF GROUP A STEPTOCOCCI IN A NEW CHEMICALLY DEFINED MEDIUM", INFECTION AND IMMUNITY, vol. 27, no. 2, 1 February 1980 (1980-02-01), pages 444-448, XP002028774, ISSN: 0019-9567
- SOCRANSKY S. S. ET AL: "Chemically Defined Medium for Oral Microorganisms", JOURNAL OF CLINICAL MICROBIOLOGY, vol. 22, no. 2, 1 August 1985 (1985-08-01) , pages 303-305, XP055337259,
- DZINK J. L. ET AL: "AMINO ACID UTILIZATION BY FUSOBACTERIUM NUCLEATUM GROWN IN A CHEMICALLY DEFINED MEDIUM", ORAL MICROBIOLOGY AND IMMUNOLOGY, vol. 5, no. 3, 1990, pages 172-174, XP008050280, ISSN: 0902-0055
- VAN DER HOEVEN J. S. ET AL: "Competition between oral Streptococcus species in the chemostat under alternating conditions of glucose limitation and excess", FEMS MICROBIOLOGY LETTERS, vol. 31, no. 6, 1 December 1985 (1985-12-01), pages 373-379, XP023916167, ISSN: 0378-1097 [retrieved on 1985-12-01]
- MONNET C. ET AL: "Selection and properties of alpha-acetolactate decarboxylase-deficient spontaneous mutants of Streptococcus thermophilus", FOOD MICROBIOLOGY, vol. 24, no. 6, 4 April 2007 (2007-04-04), pages 601-606, XP022014444, ISSN: 0740-0020, DOI: 10.1016/J.FM.2007.01.004

## Description

The present invention relates to chemically defined culture media comprising at least glutamine, cysteine and/or cystine, adenine, guanine, aminobenzoic acid, nicotinamide adenine dinucleotide and an iron(III) salt for the detection of a broad range of microorganisms selected from prokaryotes, yeasts and fungi.

Complex, general purpose media have been available for the growth and culture of bacteria, yeast and moulds since the nineteenth century. The process for manufacturing these media and the main components used has surprisingly changed very little in its fundamental nature since this time. The main basis of these microbiological media are the peptones which they contain.

The peptone(s) employed are designed to be present in combinations and at concentrations which allow growth of a very broad variety of bacteria, yeast and moulds. Thus, mixtures of these peptones are a biochemically rich and well balanced nutrient source and are usually complemented by the addition of salts, buffers and other raw materials necessary to optimize speedy growth and/or select for microorganisms with particular biochemical properties. In addition, the solid form of the medium is generally well soluble in water. After dissolving the medium is sterilisable and can be provided in a suitably convenient container to prevent contamination but at the same time allow in oxygen, if this is required, after inoculation with prokaryotic, yeast and/or fungal cells. Subsequently, the container with medium and cells is incubated at a suitable temperature and for a suitable time to allow determination (or absence) of cell growth.

However, only a careful selection of peptone types by highly experienced individuals familiar with the art will yield a medium with an adequate nutritional basis broad enough and sufficiently balanced in its biochemical composition to enable the cultivation of a very wide range of prokaryotes as well as yeasts and fungi. Peptone quality varies between peptone types, between different manufacturers of the same peptone, varies between different grades from the same manufacturer and even varies significantly between batches of the same grade from a single manufacturer. However, the biochemical nature of this varying quality is poorly understood, more especially so at the batch-to-batch variation level. However, the biochemical variation is certainly in part due to the varying quality of the natural raw materials used to manufacture peptones. Thus, not every batch of peptones is always suitable for use in particular media recipes, due to nutritional deficiencies or nutritional imbalances in such raw materials and hence each batch must be carefully selected for a particular medium to enable growth of a very wide range of prokaryotes and eukaryotes, i.e. support acceptable growth of a wide variety of bacteria, yeast and moulds.

The precise chemical composition of the peptones is not known. This means the critical nutritional factors provided by such natural raw materials resulting in cell growth promotion is understood up to a point but not well. Poorly understood and equally difficult to control are the negative physico-chemical interactions of such raw materials within a medium which cause it to precipitate, for example. Precipitation is an undesirable property of media since it optically hides microorganism growth, that is to say ideally, most media should be clear. The complexity of the problem increases when one peptone is mixed with other peptone types in addition to the salts, buffers and other components typically present in a traditional medium. The manufacture of well-balanced, general growth media is a matter of trial, error and long experience. Batch-to-batch variation of the individual peptones added must be adequately controlled such that batches unsuitable to yield the full range of desired cellular growth of the test prokaryotic (bacterial) and yeast and fungal strains, whether alone or in combination with other peptone types, can be eliminated from the production processes to manufacture such media.

Even after these measures such general media for the growth of a very wide range of cell strains are not necessarily sufficiently biochemically balanced or nutritious to grow all the bacteria, yeast and fungal strains tested in a broad test panel typically used to confirm media suitability for environmental monitoring applications. On the one hand certain cell types are very sensitive to the correct balance of biochemicals offered in the medium. On the other hand, and in addition, particularly very fastidious cells need extra supplements like fresh blood or blood extracts to boost their growth performance. However, even these media do not have a sufficient nutrient composition for certain very highly fastidious cell types and need a further addition of supplement(s) to support growth or to speed up growth in order to make them effective for their use for a broad range of prokaryotic and yeast and fungal cells typically found in environmental samples.

Lately, chemically defined cell culture media have been developed. However, to date, producers of chemically defined media for the culture of microorganisms have concentrated on supporting the growth of either prokaryotes (e.g. bacteria) or eukaryotes (e.g. yeasts or fungi or insect or mammalian cells) most often of a particular selection of microorganisms.

WO 2007/135385 for example discloses a chemically defined medium for the growth of bacteria, especially Neisseria species.

GB 2464203 discloses a chemically defined medium for the enumeration of Campylobacter.

I. Van de Rijn et al., Infection and Immunity, Vol. 27, No. 2, February 1980, p. 444-448, disclose a chemically defined medium for growing fastidious *Streptococci.*

WO 2009/026635 discloses a cell culture medium for growing *Streptococcus.*

S. Socransky et al., Journal of Clinical Microbiology, Vol. 22, No. 2, 1 August 1985, pages 303-305 disclose a cell culture medium for growing and detecting fastidious oral microorganisms.

WO 2014/019696 and WO 01/44278 disclose media supplements containing glutamine, cystine, cysteine, adenine, guanine, PABA, NAD and iron (II) salts for culturing certain microorganisms.

The aim of the present invention is thus to provide cell culture media based on chemically defined raw materials that offer a foundation for growing a very broad range of microorganisms, selected from a broad range of prokaryotic as well as yeast and fungal species comparable to the well-known peptone and/or extract-based media for example, covering the needs of growing the prokaryotic, yeast and fungal cells typically found in diverse samples, such as environmental samples, samples from food and beverage industry, pharmaceutical samples or clinical samples.

It has been found that a general, non-specific, chemically defined growth medium capable of supporting growth of a very broad range of prokaryotes, yeasts and fungi typically found in various samples, with growth potential essentially the same as traditional peptone-based media, can be provided if certain mandatory components are present in the cell culture medium and are combined with other typical media components. The medium not only fulfils an appropriate biochemical balance to grow the majority of typical isolates, prokaryote, yeast and fungal but, at the same time fulfils the requirements for supporting the growth of fastidious prokaryotic, yeast and fungal cells. Concomitantly, such media also typically enable a much speedier growth of yeast and moulds than traditional peptone-based complex media.

Consequently, the present invention is directed to a method for culturing prokaryotes, yeasts and fungi in a chemically defined cell culture medium, characterized in that the cell culture medium comprises 0.01 to 10 g/L glutamine, 0.01 to 3 g/L cysteine and/or cystine, 0.1 to 300 mg/L adenine, 0.01 to 100 mg/L guanine, 0.01 to 10 g/L aminobenzoic acid, nicotinamide adenine dinucleotide and 0.01 to 100 mg/L of an iron(III) salt.

In a preferred embodiment the cell culture medium comprises one or more saccharide components, one or more amino acids, one or more vitamins or vitamin precursors, one or more salts, one or more buffer components, one or more co-factors and one or more nucleic acid components.

In another preferred embodiment, the cell culture medium comprises a gelling agent.

In another embodiment, the cell culture medium further comprises at least one chromogenic or fluorogenic substrate.

In a preferred embodiment, the prokaryotes comprise one or more of the following strains: Staphylococcus aureus, Bacillus subtilis, Escherichia coli, Streptococcus pyogenes, Pseudomonas aeruginosa.

In a preferred embodiment, the yeasts and fungi comprise one or more of the following strains: Candida albicans, Aspergillus brasiliensis and Saccharomyces cerevisiae.

In a preferred embodiment, the prokaryotes, yeasts and fungi comprise at least one fastidious strain.

The present invention is further directed to a method for simultaneously, in one assay, detecting prokaryotes, yeasts and fungi in a sample by
a) incubating the sample in a chemically defined cell culture medium comprising 0.01 to 10 g/L glutamine, 0.01 to 3 g/L cysteine and/or cystine, 0.1 to 300 mg/L adenine, 0.01 to 100 mg/L guanine, 0.01 to 10 g/L aminobenzoic acid, nicotinamide adenine dinucleotide and 0.01 to 100 mg/L of an iron(III) salt
b) detecting the presence of the prokaryotes, yeasts and fungi.

In a preferred embodiment, the incubation in step a) is performed for less than 30 hours.

In a further preferred embodiment, the presence of the prokaryotes, yeasts and fungi is detected via their growth, optically, for example but not limited to a presence of turbidity and/or colony formation and/or via a change in color for example, but not limited to, a color induced by a pH shift due to the presence of the prokaryotes, yeasts and/or fungi.

In addition, serological tests and/or biochemical tests may be employed to confirm microbial presence using stains or dyes (like chromogens and/or fluorogens) and/or by using enzymatic tests and/or by identifying presence of microorganisms using PCR or related genetic techniques, for example, but not limited to blotting techniques, and/or microscopic examination and/or detection of ATP and/or its derivatives in a bioluminescence test and/or massively parallel sequencing and/or flow cytometry. Many other techniques are known to microbiologists. Note that a pre-concentration step may be added, for example, but not limited to, centrifugation and/or filtration in order to enable an easier and/or faster identification of microbial presence/absence and/or to aid identification.

The present invention is further directed to a chemically defined culture medium supplement comprising 0.01 to 10 g/L glutamine, 0.01 to 3 g/L cysteine and/or cystine, 0.1 to 300 mg/L adenine, 0.01 to 100 mg/L guanine, 0.01 to 10 g/L aminobenzoic acid, nicotinamide adenine dinucleotide and 0.01 to 100 mg/L of an iron(III) salt.

The present invention is further directed to the use of a chemically defined cell culture medium comprising glutamine, cysteine and/or cystine, adenine, guanine, aminobenzoic acid, nicotinamine adenine dinucleotide and an iron(III) salt as defined above for simultaneously culturing prokaryotes, yeasts and fungi.

Preferably, the chemically defined cell culture medium is used for bioburden, sterility, environmental or media fill testing.

It was found that a chemically defined cell culture medium comprising glutamine, cysteine and/or cystine, adenine, guanine, aminobenzoic acid, nicotinamide adenine dinucleotide and an iron(III) salt allows to generate a universal chemically-defined and fully synthetic culture medium promoting the rapid growth of diverse prokaryotic, yeast and fungal microorganisms as mixed or as axenic cultures.

A cell culture is any setup in which cells are cultured.

A cell culture can be performed in any container suitable for the culture of cells, such as a petri dish, contact plate, bottle, tube, well, vessel, bag, flask and/or tank. Typically the container is sterilized prior to use. Culturing is typically performed by incubation of the cells in an aqueous culture medium under suitable conditions such as suitable temperature, osmolality, aeration, agitation, etc. which limit contamination with foreign microorganisms from the environment. A person skilled in the art is aware of suitable incubation conditions for supporting or maintaining the growth/culturing of cells.

A cell culture medium (synonymously used: culture medium) according to the present invention is any mixture of components which maintains and/or supports the *in vitro* growth of cells and/or supports a particular physiological state. It is also suitable for pre-enrichment cultures as well as for use as a maintenance medium.

It is a chemically defined medium. The cell culture medium can comprise all components necessary to maintain and/or support the *in vitro* growth of cells or be used for the addition of selected components in combination with or not in combination with further components that are added separately (media supplement). Preferably, the cell culture medium comprises all components necessary to maintain and/or support the *in vitro* growth of cells.

The cell culture media according to the present invention are designed to be suitable to grow or maintain/support the growth of prokaryotic cells like bacterial cells as well as yeast and fungal cells. Also disclosed are cell culture media designed to be suitable to grow or maintain/support the growth of algae, plant, insect and/or mammalian cells and, optionally, archaea. The prokaryotes, yeasts and fungi are in the following also called microorganisms or cells.

A fastidious microorganism is a microorganism that will grow only if a number of special nutrients are present in its cell culture medium. Examples of fastidious microorganisms are *Legionella* species, *Brucella* species, *Francisella tularensis, Leptospira* species, *Borrelia burgdorferi, Bartonella* species, and *Bordetella* species.

Microorganisms whose growth shall be maintained or supported by the methods and media of the present invention are typically found in:
- environmental samples during environmental monitoring of pharmaceutical relevance
- samples obtained from raw materials, intermediates and/or finished goods of pharmaceutical relevance
- clinical samples during hospital examinations
- veterinary samples
- water samples for examination of drinking and/or waste water and/or swimming pool water
- food samples for the examination of microbial contamination
- cosmetic samples

Examples of cells of which growth is maintained/ supported/ detected by the media and methods according to the present invention are:
- bacteria:
   *Achromobacter sp.* wild type
   *Acinetobacter Iwoffii* (ATCC® 17925™)
   *Bacillus clausii* (ATCC® 700160)
   *Bacillus halodurans* wild type
   *Bacillus okuhidensis* wild type
   *Bacillus pumilus* wild type
   *Bacillus subtilis* (ATCC® 6633™)
   *Bacillus subtilis* wild type
   *Burkholderia sp.* wild type
   *Clostridium sporogenes* (ATCC® 11437™)
   *Clostridium sporogenes* (ATCC® 19404™)
   *Corynebacterium tuberculostearicum* wild type
   *Escherichia coli* (ATCC® 25922™)
   *Escherichia coli* (ATCC® 8739™)
   *Kocuria rhizophila* (ATCC® 9341™)
   *Leifsonia sp.* wild type
   *Methylobacterium extorquens* (ATCC® 43645™)
   *Methylobacterium extorquens* (NBRC 15911)
   *Methylobacterium fujisawaense* wild Type
   *Methylobacterium mesophilicum* (ATCC® 29983™)
   *Methylobacterium ssp.* wild type
   *Micrococcus luteus* (ATCC® 10240™)
   *Micrococcus lylae* wild type
   *Paenibacillus lautus* wild type
   *Pantoea sp.* wild type
   *Propionibacterium acnes* (ATCC® 6919™)
   *Pseudomonas aeruginosa* (ATCC® 9027™)
   *Ralstonia pickettii* (ATCC® 27511™)
   *Ralstonia pickettii* wild type
   *Salmonella typhimurium* (ATCC® 14028™)
   *Serratia marcescens* wild type
   *Sphingomonas parapaucimobilis* wild type
   *Sphingomonas paucimobilis* (ATCC® 29837™)
   *Sphingomonas paucimobilis* wild type
   *Staphylococcus aureus* (ATCC® 25923™)
   *Staphylococcus aureus* (ATCC® 6538™)
   *Staphylococcus epidermidis* (ATCC® 12228™)
   *Staphylococcus epidermidis* wild type
   *Staphylococcus hominis* (ATCC® 27844™)
   *Stenotrophomonas maltophilia* (ATCC® 13637™)
   *Streptococcus pyogenes* (ATCC® 12344™)
   *Streptococcus pyogenes* (ATCC® 21059™)
- yeasts:
   *Candida albicans* (ATCC® 10231™)
   *Debaryomyces hansenii* (DSM 3428)
   *Exophiala sp.* wild type
- moulds:
   *Aspergillus brasiliensis* (ATCC® 16404™)
   *Aspergillus brasiliensis* wild type
   *Aspergillus sydowii* (DSM 63373)
   *Penicillium commune* (ATCC® 10428™)

For example, the chemically defined culture medium according to the present invention may support the growth of yeasts and fungi, and of prokaryotes like Gram-positive microorganisms and Gram-negative microorganisms, such as human skin contaminants, water contaminants, yeast and mould, e.g. *Bacillus subtilis, Bacteroides vulgatus, Clostridium sporogenes, Propionibacterium acnes, Staphylococcus aureus, Apergillus brasiliensis, Candida albicans, Escherichia coli, Methylobacterium extorquens, Methylobacterium fujisawaense, Methylobacterium mesophilicum, Pseudomonas aeruginosa, Paenibacillus lautus, Ralstonia pickettii, Staphylococcus epidermidis.*

Chemically defined cell culture media are cell culture media comprising of chemically well characterized 'defined' raw materials. This means that the chemical composition of all the chemicals used in the media is known. The chemically defined media do not comprise of chemically ill-defined substances like chemically ill-defined yeast, animal or plant tissues; they do not comprise peptones, feeder cells, serum, ill-defined extracts or digests or other components which may contribute chemically poorly defined proteins and/or peptides and/or hydrolysates to the media. Chemically undefined, ill-defined or poorly defined chemical components are those whose chemical composition and structure is not well known, are present in poorly defined and varying composition or could only be defined with enormous scientific experimental effort. In some cases the chemically defined medium may comprise proteins or peptides which are chemically defined - one example is insulin (see others below).

A powdered cell culture medium or a dry powder medium or a dehydrated culture medium is a cell culture medium typically resulting from a milling process or a lyophilisation process. That means the powdered cell culture medium is typically a finely granular, particulate medium - not a liquid medium. The term "dry powder" may be used interchangeably with the term "powder;" however, "dry powder" as used herein simply refers to the gross appearance of the granulated material and is not intended to mean that the material is completely free of complexed or agglomerated solvent unless otherwise indicated. A powdered cell culture medium can also be a granulated cell culture medium, e.g. dry granulated by roller compaction or wet granulated by fluidized bed spray granulation. Such a medium can also be prepared by spray drying.

The media of the present invention for supporting the general growth of prokaryotes, yeasts and fungi show comparable (visible) growth characteristics to standard media used for this purpose. Thus they typically
a. contain a balance of biochemicals to promote cell growth of the majority of cells which tend to be detected
   i) contain sufficient specific biochemicals to supply the needs of a very wide range of prokaryotes, yeasts and fungi but at the same time
   ii) not contain concentrations of specific biochemical which may be so high as to significantly inhibit the growth of certain sensitive cell strains
b. contain a rich nutrient base suitable to bridge the auxotrophic gaps present in many prokaryotic, yeast and fungal species
c. contain certain complex biochemicals able to feed cells such that growth is not delayed unnecessarily by extensive de novo enzyme synthesis . Such undesirable, extensive de novo enzyme synthesis can lead to a
   i) significantly extended lag phase or
   ii) death of the cells since they are not able to recover sufficient metabolic activity to overcome critical cellular damage and/or balance out excessive cellular anabolic activity.

A cell culture medium which comprises all components necessary to maintain and/or support the *in vitro* growth of cells typically comprises at least one or more saccharide components, one or more amino acids, one or more vitamins or vitamin precursors, one or more salts, one or more buffer components, one or more co-factors and one or more nucleic acid components (nitrogenous bases) or their derivatives. It may also comprise chemically defined biochemicals such as recombinant proteins, e.g. rlnsulin, rBSA, rTransferrin, rCytokines etc. (see above).

The media may also comprise sodium pyruvate, highly purified and hence chemically well-defined extracts, fatty acids and/or fatty acid derivatives and/or pluronic product components (block copolymers based on ethylene oxide and propylene oxide) in particular Poloxamer 188 sometimes called Pluronic F 68 or Kolliphor P 188 or Lutrol F 68 and/or surface active components such as chemically prepared non-ionic surfactants. One example of a suitable non-ionic surfactants are difunctional block copolymer surfactants terminating in primary hydroxyl groups also called poloxamers, e.g. available under the trade name pluronic ® from BASF, Germany. Such pluronic product components are in the following just called pluronic. Chelators, hormones and/or growth factors may also be added.

Other components it may comprise of are the pure compounds, salts, conjugates, and/or derivatives of lactic acid, thioglycollic acid, thiosulphates, tetrathionate, diaminobutane, myo-inositol, phosphatidylcholine (lecithin), sphingomyelin, iron containing compounds (including compounds with iron sulphur clusters), uric acid, carbamoyl phosphate, succinic acid, thioredoxin(s), orotic acid, phosphatidic acid, polyamines (such as putrescine, spermidine, spermine and/or cadaverine), triglycerides, steroids (including but not limited to cholesterol), metallothionine, oxygen, glycerol, urea, alpha-ketoglutarate, ammonia, glycerophosphates, starch, glycogen, glyoxylate, isoprenoids, methanol, ethanol, propanol, butanol, acetone, lipids (including but not limited to those in micelles), tributyrin, butyrin, cholic acid, desoxycholic acid, polyphosphate, acetate, tartrate, malate and/or oxalate.

Saccharide components are all mono- or di-saccharides, like glucose, galactose, ribose or fructose (examples of monosaccharides) or sucrose, lactose or maltose (examples of disaccharides) or derivatives thereof like sugar alcohols. Saccharide components may also be oligo- or polysaccharides.

Examples of amino acids are particularly the proteinogenic amino acids, especially the essential amino acids, leucine, isoleucine, lysine, methionine, phenylalanine, threonine, tryptophan and valine, as well as the non-proteinogenic amino acids such as D-amino acids.

Tyrosine means L- or D- tyrosine, preferably L-tyrosine.

Cysteine means L- or D-cysteine, preferably L-cysteine.

Amino acid precursors and analogues are also included.

Examples of vitamins are Vitamin A (Retinol, retinal, various retinoids, and four carotenoids), Vitamin B₁ (Thiamine), Vitamin B₂ (Riboflavin), Vitamin B₃ (Niacin, niacinamide), Vitamin B₅ (Pantothenic acid), Vitamin B₆ (Pyridoxine, pyridoxamine, pyridoxal), Vitamin B₇ (Biotin), Vitamin B₉ (Folic acid, folinic acid), Vitamin B₁₂ (Cyanocobalamin, hydroxycobalamin, methylcobalamin), Vitamin C (Ascorbic acid) (including phosphates of ascorbic acid), Vitamin D (Ergocalciferol, cholecalciferol), Vitamin E (Tocopherols, tocotrienols) and Vitamin K (phylloquinone, menaquinones). Vitamin precursors and analogues are also included.

Examples of salts are components comprising inorganic ions such as bicarbonate, calcium, chloride, magnesium, phosphate, potassium and sodium or trace elements such as Co, Cu, F, Fe, Mn, Mo, Ni, Se, Si, Ni, Bi, V and Zn. Examples are copper(II) sulphate pentahydrate (CuSO₄·5 H₂O), sodium chloride (NaCI), calcium chloride (CaCl₂·2 H₂O), potassium chloride (KCI), iron(II)sulphate, sodium phosphate monobasic anhydrous (NaH₂PO₄), magnesium sulphate anhydrous (MgSO₄), sodium phosphate dibasic anhydrous (Na₂HPO₄), magnesium chloride hexahydrate (MgCl₂·6 H₂O), zinc sulphate heptahydrate (ZnSO₄·7 H₂O).

Examples of buffers are carbonate, citrate, phosphate, HEPES, PIPES, ACES, BES, TES, MOPS and TRIS.

Examples of cofactors are compounds, salts, complexes and/or derivatives of thiamine, biotin, vitamin C, calciferol, choline, NAD/NADP (reduced and/or oxidized), cobalamin, vitamin B12, flavin mononucleotide and derivatives, flavin adenine dinucleotide and derivatives, glutathione (reduced and/or oxidized and/or as dimer), haeme, haemin, haemoglobin, ferritin, nucleotide phosphates and/or derivatives (e.g. adenosine phosphates), coenzyme F420, S-adenosyl methionine, coenzyme B, coenzyme M, coenzyme Q, acetyl Co-A, molybdopterin, pyrroloquinoline quinone, tetrahydrobiopterin.

Nucleic acid components are the nucleobases, like cytosine, guanine, adenine, thymine, uracil, xanthine and/or hypoxanthine, the nucleosides like cytidine, uridine, adenosine, xanthosine, inosine, guanosine and thymidine, and the nucleotides such as adenosine monophosphate or adenosine diphosphate or adenosine triphosphate, including but not limited to the deoxy- and/or phosphate derivatives and/or dimers, trimers and/or polymers thereof, like RNA and/or DNA.

Compounds may be added which improve the physico-chemical properties of the media, like but not limited to, increasing clarity and/or solubility of the media and/or one or more of its components, without significantly negatively affecting the cell growth properties at the concentrations used. Such compounds include but are not limited to chelating agents (e.g. EDTA), antioxidants, detergents, surfactants, emulsifiers (like polysorbate 80), neutralising agents, (like polysorbate 80), micelle forming agents, micelle inhibiting agents and/or polypropylene glycol, polyethylene alcohol and/or carboxymethylcellulose.

The medium typically contains carbohydrates such as sugars and/or sugar mixtures and/or sugar dimers and/or sugar polymers and/or their derivatives. Typically, glucose and/or lactose and/or galactose can be the main carbohydrate sugar components. Glucose is usually included in the medium at a concentration of 0.001 mM to 250 mM in the aqueous medium solution, more preferably 1 mM to 100 mM, even more preferably 5 mM to 50 mM.

Typically, the medium comprises each amino acid in a range from 10 mg to 3 g per liter, preferably in a range from 40 mg to 1 g per liter.

The medium typically comprises vitamins. A typical amount of a vitamin in the medium is in the range of 5 µg to 10 mg per liter, preferably in the range of 50 µg to 6 mg per liter.

Typically, the medium comprises salts. The amount of one type of salt is typically in the range of 2 µg to 5 mg per liter, preferably in the range of 10 µg to 1.5 mg per liter. Specific salts may also be present in much higher amounts; the concentration of NaCl can for example be up to 5 g per liter.

The typical amount of a nucleic acid comprised in the medium is in the range of 0.5 to 10 mg per liter, preferably in the range of 1 to 5 mg per liter.

The medium typically contains all the proteogenic amino acids (and/or their derivatives and/or their conjugates and/or dimers (pure and/or mixed) thereof). It should be noted that the concentrations of the components in the solid medium may differ significantly to those practically measured after dissolution. This is because, for example, certain amino acids can react in the aqueous medium with other components to form products which then indirectly contain the amino acids by which the pure amino acid in solution is thereby depleted. This process may also occur to other easily reactive constituents, for example, but not limited to vitamin C and/or indeed the amino acids may react with each other or with themselves. This process may be an oxidative process dependent on oxygen concentrations and the presence of trace and/or ultra trace elements, in particular the transition metal ions like those of copper and/or iron added directly as components and/or present as contaminants.

Other defined components may also be added to aid detection or identification of microorganisms like indicators.

The chemically defined culture medium according to the present invention can further comprise at least one chromogenic or fluorogenic substrate. Fluorogenic substrates are complex molecules which, on contact with enzymes synthesized by microorganisms, are cleaved and become fluorescent. The fluorescence emitted is detectable visually and/or with an analytical instrument like a spectrophotometer by illuminating the growth medium using radiation in the UV or visible spectrum. Examples of fluorogenic substrates are fluorescein derivatives (CFA, CFDA), methylumbelliferone derivatives or the Aldols™ (developed by the company Biosynth).

Chromogenic substrates are substrates that change their color when they are modified e.g. by a specific enzyme of a microorganism. Examples of chromogenic substrates are ONPG (Ortho-nitrophenyl-p-D-galactopyranoside) or X-Gal (5-bromo-4-chloro-3-indolyl-beta-D-galactopyranoside). In this case the medium color after the growth of certain microorganisms of interest will change color and be indicative of such microorganisms.

A typically suitable liquid cell culture medium has a typical composition of 2 to 50 g/L, more preferably 5 to 30 g/L. A medium with a gelling agent has typically an additional weight due to the gelling agent of between 1 and 50 g/L, more preferably between 2 and 30 g/L.

The osmolality of the medium is typically between 50 mOsm and 1000 mOsm, more preferably between 150 mOsm and 500 mOsm.

Since the suitable cell culture media are free from animal and plant derived peptones they represent a highly reduced likelihood of being a source of adventitious agent contamination at their point of use such as in pharmaceutical applications of environmental testing. Such infectious contaminants which will be very significantly reduced in their presence include, but are not limited to:
- proteinaceous contaminants like bovine spongiform encephalopathy (BSE),
- viral contaminants like minute virus of mice (MVM),
- heat resistant spore forming prokaryotes,
- eukaryotic contaminants,
- cells known to be capable or penetrating membrane pores during sterile filtration due to their extremely small size under certain conditions such as mycoplasma like Burkholderia cepacia.

Disclosed is a chemically defined cell culture medium which comprises in addition to a typical chemically defined cell culture medium e.g. for the culture of CHO cells the following components and/or their salts and/or their derivatives and/or conjugates and/or dimers (pure or mixed).

**Table 1**

| Component | Typically | Preferred | More Preferred | Even More |
|---|---|---|---|---|
| | | | | |
| Glutamine | 0,001 - 50 g/L | 0,01 - 10 g/L | 0,02 - 2 g/L | 0,1 g/L |
| Cysteine and/or Cystine | 0,001 - 10 g/L | 0,01 - 3 g/L | 0,05 - 1 g/L | 0,5 g/l |
| Adenine | 0,01 - 1000 mg/L | 0,1 - 300 mg/L | 2 - 50 mg/L | 10 mg/L |
| Guanine | 0,001 - 500 mg/L | 0,01 - 100 mg/L | 0,05 - 5 mg/L | 0,5 mg/L |
| Aminobenzoic Acid | 0,001 - 100 g/L | 0,01 - 10 g/L | 0,02 - 2 g/L | 0,1 g/L |
| Nicotinamide adenine dinucleotide | 0,001 - 500 mg/L | 0,1 - 100 mg/L | 0,5 - 25 mg/L | 2,5 mg/L |
| Fe concentration e.g. from Fe(III) nitrate | 0,001 - 500 mg/L | 0,01 - 100 mg/L | 0,05 - 5 mg/L | 0,5 mg/L |

Disclosed examples of suitable iron salts are ammonium iron(II) sulfate hexahydrate, iron(II) sulfate heptahydrate or iron(III) nitrate nonahydrate. Chelates of iron may also be used and/or iron containing proteins or peptides.

Cysteine and/or Cystine might be added as L-cysteine, L-cysteine hydrochloride, L-cystine, N-acetyl-cysteine.

Glutamine may be added as the pure compound and/or I-analyl-I-glutamine or the hydrochloric acid salts, for example.

Adenine and guanine can be added as the pure compounds or as the hydrochloric acid salts, for example.

Aminobenzoic acid can be added as the pure chemical or as a salt for example with sodium.

Nicotinamide adenine dinucleotide can be added as the oxidised or reduced for and/or as the phosphate derivative and/or as other derivatives known to those in the art.

The cell culture medium can be a dry powder medium, a liquid medium or a semi-solid medium. In case of a semi-solid medium, the medium comprises beside the chemically defined components a gelling agent. An example of a suitable gelling agent is agar-agar and/or agarose.

Powdered cell culture media are preferably produced by mixing all components and milling them. The mixing of the components is known to a person skilled in the art of producing dry powdered cell culture media by milling. Preferably, all components are thoroughly mixed so that all parts of the mixture have nearly the same composition. The higher the uniformity of the composition, the better the quality of the resulting medium with respect to homogeneity.

The milling can be performed with any type of mill suitable for producing powdered cell culture media. Typical examples are ball mills, pin mills, fitz mills or jet mills. Preferred is a pin mill, a fitz mill or a jet mill, very preferred is a pin mill. Even more preferred is a pin mill in which the milling chamber is cooled with nitrogen.

Preferably, all components of the mixture which is subjected to milling are dry. This means, if they comprise water, they only comprise water of crystallization but not more than 10%, preferably not more than 5% most preferred not more than 2% by weight of unbound or uncoordinated water molecules. They may also contain liquids bound within the media such that the powder properties are not significantly impacted.

Dry powder cell culture media can also be used as compactates to facilitate handling. Typically compacted media have good dissolving properties and are easier to handle due to reduced dust formation.

The powder media are preferably compacted in a roll press.

For use of the dry powdered media a solvent, preferably water (most particularly distilled and/or deionized water or purified water or water for injection) or an aqueous buffer is added to the media and the components are mixed until the medium is totally dissolved in the solvent.

The solvent may also comprise saline, soluble acid or base ions providing a suitable pH range (typically in the range between pH 1 and pH 10), stabilizers, surfactants, preservatives, and alcohols or other polar organic solvents as well as gelling agents for the production of semi-solid media.

The media are preferably treated to significantly reduce bioburden load prior to use to such a level that biological contaminants are extremely rarely present in the final, treated medium prior to use. This treatment can be preferably performed in the liquid state by filtration and/or by heat treatment (e.g. 121°C for 15 minutes) and/or by UV treatment.

The pH of the dissolved medium prior to addition of cells is typically between pH 2 and 12, more preferable between pH 4 and 10, even more preferably between pH 6 and 8 and most preferable between pH 6.5 to 7.5 and ideally between pH 7.0 to 7.5.

The culture medium according to the present invention can be used under aerobic as well as anaerobic growth conditions. The person skilled in the art is familiar with the respective measures to be taken for aerobic or anaerobic growth. Typically, for anaerobic culture conditions oxygen is removed, for example by an additive that reduces or preferably eliminates the oxygen in the aqueous medium and/or by a physical means under vacuum and/or by boiling out the oxygen gas. If an additive is used then preferably, the additive reacts with the oxygen dissolved in the medium to chemically remove is and thus create an anaerobic environment. By whichever means then the container containing the oxygen-depleted medium should prevent fresh oxygen from intruding into the culture medium. The additive may comprise a reducing agent, or may also be an oxygen absorber or scavenger such as a palladium catalyst, or an enzyme, e.g. a mono- and/or di-oxygenase, and/or succinate.

It has been found that chemically defined cell culture media comprising glutamine, cysteine and/or cystine, adenine, guanine, aminobenzoic acid, nicotinamide adenine dinucleotide and an iron(III) salt support the growth of prokaryotes, yeasts and fungi. The media can vary broadly in their composition as long as they comprise said components in amounts as claimed.

Consequently, glutamine, cysteine and/or cystine, adenine, guanine, aminobenzoic acid, nicotinamide adenine dinucleotide and an iron(III) salt can be added to and/or incorporated into a broad variety of chemically defined cell culture media to improve the applicability of those media for the purpose of growing a broad range of prokaryotes, yeasts and fungi.

The present invention is therefore further directed to a cell culture medium supplement comprising glutamine, cysteine and/or cystine, adenine, guanine, aminobenzoic acid, nicotinamide adenine dinucleotide and an iron(III) salt in amounts as claimed, and indicated as preferred amounts in Table 1.

According to the invention, the cell culture medium supplement contains 0.01 to 10 g/L glutamine, 0.01 to 3 g/L cysteine and/or cystine, 0.1 to 300 mg/L adenine, 0.01 to 100 mg/L guanine, 0.01 to 10 g/L aminobenzoic acid, nicotinamide adenine nucleotide and 0.01 to 100 mg/L of an iron(III) salt.

The medium supplement of the present invention is typically produced and used in the same way as the cell culture media of the present invention. The supplement might be added to a dry powder medium and be thoroughly mixed with this medium prior to dissolution and use. It might also be dissolved in water or an aqueous buffer like the cell culture medium itself and mixed with the dissolved cell culture medium afterwards subsequent to or prior to treatment to remove biological contaminant, for example by filtration.

Disclosed examples of chemically defined media that can be supplemented with the medium supplement of the present invention are those used for the purpose of growing mammalian and/or insect cells.

Especially suitable media to be supplemented with the supplement of the present invention are cell culture media formulations for CHO cells. Those are well documented in the literature and several are commercially available e.g. from Merck KGaA (Darmstadt, Germany).

Typical defined media bases (without any addition of peptones or sodium bicarbonate) for the culture of culture of CHO cells can be found under:
- Formulation for F-12K Medium, ATCC® 30-2004
- Composition of serum-free medium CHO-T1-SF (from: Journal of Biotechnology, 2004, 108, p 279-292, Schroder et al., "Serum- and protein-free media formulations for the Chinese hamster ovary cell line DUKXB11").
- US Patent Application No. US 2006/0148074 A1 to Gorfien et al., date of publication 06.07.2006, "Serum-free mammalian cell culture medium, and uses thereof".

Further disclosed is a method of preparing a chemically defined cell culture medium that supports the culture/growth of prokaryotes and eukaryotes by adding a cell culture medium supplement comprising glutamine, cysteine and/or cystine, adenine, guanine, aminobenzoic acid, nicotinamide adenine dinucleotide and an iron salt to a chemically defined mammalian and/or insect cell culture medium.

The gist of the present invention is to provide a cell culture medium that is chemically defined and supports the rapid culture/growth of a very broad range of prokaryotes, yeasts and fungi. Since the medium has an exact chemical formulation batch-to-batch production exhibits minimal variation and reproducibility can be guaranteed. Since the media are substantially free from animal and plant derived peptones they represent a highly reduced likelihood of being a source of adventitious agent contamination at their point of use such as in pharmaceutical applications or environmental testing.

The present invention is further directed to a method for culturing microorganisms in a culture medium as defined above, as well as to the use of a cell culture medium according to the present invention for the culturing of microorganisms. The microorganisms are selected from the group consisting of bacteria, yeasts and fungi.

Typically, the medium is placed in a suitable container and inoculated with the microorganisms or a sample potentially comprising said microorganisms. Suitable containers are defined above. A sample can be any liquid, gaseous or solid entity that can be contacted with the cell culture medium. The sample can be a surface that is e.g. contacted with a contact plate comprising the cell culture medium. It can for example be a swap that is contacted with the cell culture medium or any liquid or solid that is put in or onto the medium.

The temperature of incubation of the medium to allow growth of cells is typically between 0 °C and 100 °C, more typically between 15 °C and 50 °C, still more typically between 20 and 45°C. The samples may for example be incubated at room temperature (around 20°), e.g. at 22.5°C or at 32.5°C.

Generally, after inoculation, the medium is incubated for a period of time to enable some growth of the microorganisms so that they can be easily detected. For traditional methods, this time can range from a minimum of hours to weeks. Generally, the incubation time is between about 1 and 14 days. The media manufactured with this method enable detection of certain microorganisms after less than 24 hours, even of microorganisms which traditionally take two days to grow. In particular the yeasts and moulds show a growth rate which is unusually fast compared to traditional media manufactured with peptones.

The present invention is further directed to a method for simultaneously, in one assay, detecting prokaryotes, yeasts and fungi in a sample by
a) Contacting the sample with a chemically defined cell culture medium comprising glutamine, cysteine and/or cystine, adenine, guanine, aminobenzoic acid, nicotinamide adenine dinucleotide and an iron(III) salt
b) incubating the inoculated cell culture medium of step a)
c) detecting the presence of the prokaryotes, yeasts and fungi in the said cell culture medium.

Steps a) and b) are performed as described above for culturing the microorganisms. The detection in step c) can be done by any known method. Examples are visual detection of colonies formed on a semi-solid medium or of turbidity in a liquid media, detection of a change in colour or fluorescence resulting from chromogenic or fluorogenic substrate which is present in the medium. Growth can be discerned using pH changes resulting from biochemical transformations brought about by microorganism enzymatic activity producing organic acids, for example, or ammonium production causing the pH to rise significantly. Other methods for detection include PCR analysis. Those versed in the art are able to identify many other methods which can be used in combination with such media which are able to directly or indirectly detect the presence of microorganisms even if there is no visible growth.

The chemically defined media according to the present invention can replace the traditional complex culture media in various applications, e.g. in the biopharmaceutical or environmental field, in food and beverage industry or in diagnostics. Exemplary applications are listed in the following:

### Sterility and bioburden testing:

The media according to the present invention can be used in sterility and bioburden testing. Sterility testing of in-process material and final products must be demonstrated during the manufacture of pharmaceuticals and medical devices. The regular microbiological control of beverages or drinking water is also of great importance.

A typical method for testing bioburden levels or sterility of a sample is to filter a liquid sample over a membrane filter which retains the possible contaminants of the sample and incubating the filter in the culture medium. If the sample is non-filterable, the sample can directly be inoculated into the culture medium. In the event of one or more contaminants being present, the transparent medium becomes cloudy owing to the development of the cells.

Within this regard the use of a chemically defined culture medium according to the present invention is advantageous since it is preferably a clear medium in which even a low turbidity can easily be detected. One advantage is that the medium color can be more easily tuned to aid visible detection.

Filters to be used in this application have a pore size small enough to trap any microorganisms in the sample. Such filters typically have a pore size from about 0.1 µm up to 1.2 µm. Preferably, the pore size is of 0.45 µm or less. The filters can be formed of any suitable material commonly used for such applications, such as regenerated cellulose, mixed cellulose esters, cellulose acetate, polyethersulfone, polyarylsulfone and polyphenylsulfone.

Holders for the filters may simply be a stainless device such as a funnel. Alternatively, disposable, pre-sterilized and transparent filter containing devices can be used, such as the Steritest™ EZ device (Merck Millipore), which is a closed device allowing to conduct the entire test (sampling, filtration, media addition and incubation).

### Environmental testing:

A typical method for testing for environmental levels of microbes in a facility is to filter a sample of air through a device, retaining the microorganisms on the filter. The filter can then be incubated in the culture medium according to the present invention as discussed above.

Another typical method is the use of such media in contact plates.

Particularly here the highly purified and chemically defined nature of the raw materials in the media mean that said media is significantly less likely to contain adventious agents. The surface of a contact plate composes the media in a semi-solid form. The media surface is pressed against, for example, an equipment surface in order to determine if the equipment is contaminated with microorganisms. By definition then a small amount of media is left behind on this equipment surface. This means that the finger print of the media left behind after testing on this critical surface (e.g. in a pharmaceutical environment) is slightly contaminated with media. With traditional media then the surface is subsequently contaminated with ill-defined peptones and other ill-defined raw materials. Such raw materials harbour a much higher risk of carrying with them adventitious agents such as BSE/TSE agents and viruses. Against this a chemically defined, synthetic media is a low risk option which is far less likely to harbour such adventitious agents.

### Media fill test:

Media fill tests are performed on a regular basis in order to verify that aseptic production processes are not affected by microbial contamination, such as spoilage bacteria, yeasts or moulds, e.g. in pharmaceutical or food and beverage industry. Typically, in a media fill test the entire process of production of a product is simulated with a sterile culture medium instead of the respective product. The medium is then filled in separate units which are subject to a sterility test as defined above. Again, as in the case above, the reduced risk of inadvertent contamination with adventitious agents is much lower when using a media free from ill-defined peptones/extracts which are derived from processes and have an origin based in animal tissue and/or farms.

### Pre-enrichment before specific identification:

In a further aspect the media according to the present invention can be used for pre-enrichment of microorganisms before identification. For this purpose, the sample is inoculated in the media and the microorganisms are grown for a sufficient period of time. As soon as the desired concentration is reached the sample is subjected to further treatment, such as specific microbiological identification.

A further aspect of the present invention is therefore a method of detecting microorganisms in a sample for which it is sought to determine whether it is contaminated with a living microorganism, characterized in that it comprises a step of inoculating the sample in a culture medium as defined above and a step of observing the growth of microorganisms.

The present invention is further directed to the use of a cell culture medium according to the present invention for bioburden, sterility, environmental or media fill testing.

With the present invention is could be shown that also chemically defined cell culture media are suitable for the simultaneous detection of yeasts, fungi and prokaryotes if said media comprise a certain combination of additives (glutamine, cysteine and/or cystine, adenine, guanine, aminobenzoic acid, nicotinaminde adenine dinucleotide and an iron(III) salt)

in addition to the typical ingredients of a cell culture medium. Cell culture media are known which comprise one or more of the said additives but up to now it was not recognized that chemically defined media comprising said components are especially suitable for supporting the growth of a wide range of microorganisms. The media and methods of the present invention thus provide the possibility to grow and optionally also detect such microorganisms without being forced to use a cell culture medium comprising chemically ill-defined peptones and or extracts which are known to harbor adventitious agents. The media and methods of the present invention are equally sensitive and fast as the known media and methods comprising the use of peptones.

The present invention is further illustrated by the following examples, however, without being restricted thereto. The scope of the invention is defined by the claims.

### Examples

### Test procedure for all strains:

- Filter-sterilized liquid culture media are prepared extemporaneously or stored in 125mL glass bottles in the fridge before use.
- Serial dilution of a strain solution stored in HEPES/Glycerol at -80°C is performed in 0.9% sodium chloride solution.
- Each culture medium bottle is inoculated with 20 to 50 CFU/mL.
- The inoculated bottles are incubated at 22.5°C±2.5 and 32.5°C±2.5 for 14 days maximum depending on the strain.
- Sterile bottles are incubated in parallel as control.
- Microorganism development is visually observed in each bottle.

### Example 1

Eight cell strains typically used for testing the growth potential of Tryptic Soy Agar (TSA) are selected for screening the suitability of chemically defined, synthetic medium CHO Medium (Cellvento™ CHO-100, product item number 1.00899 from Merck-Millipore, Darmstadt, Germany) supplemented with agar-agar as a substitute for said TSA. Growth parameters of CHO-S are measured compared to TSA, item number 1.05458.0500 purchased from Merck-Millipore, Darmstadt, Germany. The pre-cultures are prepared in Tryptic Soy Broth (TSB), item number 1.05459.0500 purchased from Merck-Millipore, Darmstadt, Germany in which they are grown aerobically for 24 hours at 37 °C with the exception of the Aspergillus spp. which is prepared directly by washing off and resuspending fungal spores from an agar plate. Pre-cultures are diluted in sodium chloride peptone broth and plated out using a spiral plater. The CHO-S Medium is prepared from double concentrated CHO-100 medium with adjustment of the pH to 7,3 using sodium carbonate and the solution is sterile filtrated after addition of the aqueous supplement concentrate. Separately, agar is suspended in water at double concentration (26 g/L) and autoclaved at 121 °C for 20 minutes. The agar solution is then cooled to around 46 °C and mixed with double concentrated CHO-S Medium at the same temperature. Agar plates are prepared after gentle but thorough mixing of the two solutions whilst avoiding extraneous microbial contamination. After surface inoculation the plates are incubated aerobically at 32 °C +/- 2,5 °C and scored after 24 hours.
The results are shown in Table 2.

**Table 2**

| **Test Cell Strains** | **Comparison Parameter** | **Tryptic Soy Agar** (Control) | **CHO Medium** (no supplements) | **CHO-S Medium** (with supplements) |
|---|---|---|---|---|
| | | Reading 24h | Reading 24h | Reading 24h |
| **Staphylococcus aureus ATCC 6538** | Count (CFU) | 418 | 561 | 737 |
| | % Recovery | 100% | 134% | 176% |
| | Growth | 4 | 3-4 | 3-4 |
| **Bacillus subtilis ATCC 6633** | Count (CFU) | 50 | 64 | 84 |
| | % Recovery | 100% | 128% | 168% |
| | Growth | 3 | 3 | 3 |
| **Escherichia coli ATCC 8739** | Count (CFU) | 564 | 561 | 571 |
| | % Recovery | 100% | 99% | 101% |
| | Growth | 3-4 | 3-4 | 3-4 |
| **Streptococcus pyogenes ATCC 21059** | Count (CFU) | 20 | 0 | 11 |
| | % Recovery | 100% | 0% | 55% |
| | Growth | 3-4 | 0 | 3-4 |
| **Pseudomonas aeruginosa ATCC 9027** | Count (CFU) | 701 | 524 | 693 |
| | % Recovery | 100% | 75% | 99% |
| | Growth | 3 | 4 | 4 |
| **Candida albicans ATCC 10231** | Count (CFU) | 306 | 304 | 314 |
| | % Recovery | 100% | 99% | 103% |
| | Growth | 3 | 3 | 3-4 |
| **Aspergillus brasiliensis ATCC 16406** | Count (CFU) | 25 | 15 | 27 |
| | % Recovery | 100% | 60% | 108% |
| | Growth | 1 | 1 | 1 |
| **Streptococcus pyogenes ATCC 12344** | Count (CFU) | 506 | 4 | 502 |
| | % Recovery | 100% | 1% | 99% |
| | Growth | 3-4 | 3-4 | 3-4 |

Growth is given as the relative diameter of colonies on a scale of 0 (no growth) to 5 (luxuriant growth for the respective cell strains) as read at 24 hours after incubation at 32,5 +/- 2,5 °C.

### Summary

Speed of growth, represented by colony size after 24 hours, and percent recovery, are all in good comparison with TSA but only after addition of the supplement components glutamine, cysteine/cystine, adenine, guanine, aminobenzoic acid, nicotinamide adenine dinucleotide and iron salts to the CHO medium. This is the case for almost all the bacteria tested including the gram positive organisms (both the Streptococcus pyogenes cell strains, Staphylococcus aureus and Bacillus subtilis) as well as the gram negative cell strain Pseudomonas aeruginosa. Indeed, this positive effect can also be seen for the eukaryotic cells tested: including an improvement of percent recovery of Aspergillus brasiliensis as well as a slight increase in the colony size of the Candida strain tested. Thus, both the prokaryotes and the eukaryotes tested show significant growth improvement and/or colony size to make the medium comparable with tryptic soy agar.

### Example 2

The following 49 strains of prokaryotes and eukaryotes are selected for testing as they can be environmentally relevant or are isolated from real environmental samples.

Results of positive growth, as identified visually within 14 days of incubation at the given temperatures are shown in Table 3.

Inoculum refers to the numbers of cells inoculated into the TSB or CHO S medium. The preparation of the CHO-S medium is described in Example 1.

**Table 3**

| | | **TSB** | | **CHO-S Medium** | |
|---|---|---|---|---|---|
| **Teststrain** | **Inoculum** | **22,5°C** | **32,5°C** | **22,5°C** | **32,5°C** |
| B. clausii ATCC 700160 | *10* | + | + | + | + |
| B. pumilus WT ARDS6V56 | *92* | + | + | + | + |
| B. subtilis ATCC 6633 | *39* | + | + | + | + |
| B. subtilis WT C-J | *20* | + | + | + | + |
| C. albicans ATCC 10231 | *200* | + | + | + | + |
| E. coli ATCC 8739 | *22* | + | + | + | + |
| E. coli ATCC 25922 | *35* | + | + | + | + |
| P. aeruginosa ATCC 9027 | *96* | + | + | + | + |
| S. typhimurium ATCC 14028 | *185* | + | + | + | + |
| S. aureus ATCC 25923 | *38* | + | + | + | + |
| Ral. pickettii ATCC 27511 | *59* | + | + | + | + |
| Ral. pickettii WT PQK | *26* | + | + | + | + |
| Sph. paucimobilis ATCC 29837 | *34* | + | + | + | + |
| S. aureus ATCC 6538 | *34* | + | + | + | + |
| S. epidermidis ATCC 12228 | *16* | + | + | + | + |
| S. epidermidis WT CJ | *64* | + | + | + | + |
| S. hominis ATCC 27844 | *33* | + | + | + | + |
| Steno. maltophilia ATCC 13637 | *36* | + | + | + | + |
| Strept. pyogenes ATCC 12344 | *32* | + | + | + | + |
| Ser. marcescens WT Bb | *38* | + | + | + | + |
| Acinetobacter Iwoffii ATCC 17925 | *42* | + | + | + | + |
| Aspergillus brasiliensis ATCC 16404 | | + | + | + | + |
| Aspergillus niger Umweltisolat | | + | + | + | + |
| Aspergillus sydowii DSM 63373 | | + | + | + | + |
| Penicillium commune ATCC 10428 | | + | + | + | + |
| Sphingomonas paucimobilis WT AP-W | | + | + | + | + |
| Sphingomonas parapaucimobilis WT M | | + | + | + | + |
| Pantoea sp. WT MT - 1661 | *39* | + | + | + | + |
| Achromobacter sp. WT C-2929 | *209* | + | + | + | + |
| Clostridium sporogenes ATCC 11437 | *21* | + | + | - | + |
| Clostridium sporogenes ATCC 19404 | *23* | - | + | + | - |
| Debariomyces hansenii DSM 3428 | >*50* | + | + | + | + |
| Kocuria rhizophila ATCC 9341 | *40* | + | + | + | + |
| *Methylobacterium* extorquens ATCC 43645 | *27* | + | + | + | + |
| Methylobacterium extorquens NBRC 15911 | *24* | + | - | + | + |
| Methylobacterium fujisawaense Wild Type | *54* | + | + | - | + |
| *Methylobacterium* mesophilicum ATCC 29983 | *16* | + | + | + | - |
| Methylobacterium ssp. WT CJ | *31* | + | + | + | + |
| Micrococcus luteus ATCC 10240 | *40* | + | + | + | + |
| Micrococcus ssp. lylae Rentschler Wild Type | *5* | + | + | + | + |
| Paenibacillus lautus Wild Type | *27* | + | + | + | + |
| Propionibacterium acnes ATCC 6919 | *35* | - | + | + | + |
| Streptococcus pyogenes ATCC 21059 | *30* | + | + | + | + |
| Exophiala sp. WT MT-1570 | *>50* | + | + | + | + |
| Burkholderia sp. WT C-2424 | *66* | + | + | + | + |
| Leifsonia sp. WT C-2946 | *64* | + | + | + | + |
| Corynebact. tuberculostearicum WT | *25* | + | + | + | + |
| Bacillus halodurans WT | *32* | + | + | + | + |
| Bacillus okuhidensis WT C-3745 | *42* | + | + | + | + |

The results show that all the prokaryotes and eukaryotes tested grow well in TSA and in the chemically defined cell culture medium according to the present invention.

## Claims

1. A method for the culturing of prokaryotes, yeasts and fungi in a chemically defined cell culture medium, **characterized in that** the cells are incubated in a cell culture medium comprising 0.01 to 10 g/L glutamine, 0.01 to 3 g/L cysteine and/or cystine, 0.1 to 300 mg/L adenine, 0.01 to 100 mg/L guanine, 0.01 to 10 g/L aminobenzoic acid, nicotinamide adenine dinucleotide and 0.01 to 100 mg/L of an iron(III) salt.

2. Method according to claim 1, **characterized in that** the cell culture medium comprises one or more saccharide components, one or more amino acids, one or more vitamins or vitamin precursors, one or more salts, one or more buffer components, one or more co-factors and one or more nucleic acid components.

3. Method according to one or more of claims 1 to 2, **characterized in that** the cell culture medium comprises a gelling agent.

4. Method according to one or more of claims 1 to 3, **characterized in that** the prokaryotes that are cultured comprise one or more of the following strains: Staphylococcus aureus, Bacillus subtilis, Escherichia coli, Streptococcus pyogenes, Pseudomonas aeruginosa.

5. Method according to one or more of claims 1 to 4, **characterized in that** the yeasts and fungi that are cultured comprise one or more of the following strains: Candida albicans, Aspergillus brasiliensis and/or Saccharomyces cerevisiae.

6. Method according to one or more of claims 1 to 5, **characterized in that** the prokaryotes, yeasts and fungi comprise at least one fastidious strain.

7. A method for detecting prokaryotes, yeasts and fungi in a sample by
a) Contacting the sample with a chemically defined cell culture medium comprising 0.01 to 10 g/L glutamine, 0.01 to 3 g/L cysteine and/or cystine, 0.1 to 300 mg/L adenine, 0.01 to 100 mg/L guanine, 0.01 to 10 g/L aminobenzoic acid, nicotinamide adenine dinucleotide and 0.01 to 100 mg/L of an iron(III) salt
b) Incubating the cell culture medium of step a)
c) detecting the presence of the prokaryotes, yeasts and fungi in the cell culture medium.

8. Method according to claim 7, **characterized in that** the incubation in step b) is performed for less than 30 hours.

9. Method according to claim 7 or 8, **characterized in that** the presence of the prokaryotes, yeasts and fungi is detected via their growth or via a change in color or pH induced by the prokaryotes, yeasts and fungi.

10. Method according to one or more of claims 7 to 9, **characterized in that** the medium comprises a gelling agent.

11. Use of a chemically defined cell culture medium comprising 0.01 to 10 g/L glutamine, 0.01 to 3 g/L cysteine and/or cystine, 0.1 to 300 mg/L adenine, 0.01 to 100 mg/L guanine, 0.01 to 10 g/L aminobenzoic acid, nicotinamide adenine dinucleotide and 0.01 to 100 mg/L of an iron(III) salt for culturing prokaryotes, yeasts and fungi for bioburden, sterility, environmental or media fill testing.

12. Cell culture medium supplement comprising 0.01 to 10 g/L glutamine, 0.01 to 3 g/L cysteine and/or cystine, 0.1 to 300 mg/L adenine, 0.01 to 100 mg/L guanine, 0.01 to 10 g/L aminobenzoic acid, nicotinamide adenine dinucleotide and 0.01 to 100 mg/L of an iron(III) salt.

## Patentansprüche

1. Verfahren zur Züchten von Prokaryonten, Hefen und Pilzen in einem chemisch definierten Zellkulturmedium, **dadurch gekennzeichnet, dass** die Zellen in einem Zellkulturmedium inkubiert werden, das 0,01 bis 10 g/l Glutamin, 0,01 bis 3 g/l Cystein und/oder Cystin, 0,1 bis 300 mg/l Adenin, 0,01 bis 100 mg/l Guanin, 0,01 bis 10 g/l Aminobenzoesäure, Nikotinamid-Adenin-Dinukleotid, und 0,01 bis 100 mg/l eines Eisen(III)-salzes umfasst.

2. Verfahren nach Anspruch 1, **dadurch gekennzeichnet, dass** das Zellkulturmedium eine oder mehrere Saccharidkomponenten, eine oder mehrere Aminosäuren, ein(en) oder mehrere Vitamine oder Vitaminvorläufer, ein oder mehrere Salze, eine oder mehrere Pufferkomponenten, einen oder mehrere Co-Faktoren und eine oder mehrere Nukleinsäurekomponenten umfasst.

3. Verfahren nach einem oder mehreren der Ansprüche 1 bis 2, **dadurch gekennzeichnet, dass** das Zellkulturmedium ein Geliermittel umfasst.

4. Verfahren nach einem oder mehreren der Ansprüche 1 bis 3, **dadurch gekennzeichnet, dass** die kultivierten Prokaryoten einen oder mehrere der folgenden Stämme umfassen: Staphylococcus aureus, Bacillus subtilis, Escherichia coli, Streptococcus pyogenes, Pseudomonas aeruginosa.

5. Verfahren nach einem oder mehreren der Ansprüche 1 bis 4, **dadurch gekennzeichnet, dass** die kultivierten Hefen und Pilze einen oder mehrere der folgenden Stämme umfassen: Candida albicans, Aspergillus brasiliensis und/oder Saccharomyces cerevisiae.

6. Verfahren nach einem oder mehreren der Ansprüche 1 bis 5, **dadurch gekennzeichnet, dass** die Prokaryonten, Hefen und Pilze mindestens einen anspruchsvollen Stamm umfassen.

7. Verfahren zum Nachweisen von Prokaryoten, Hefen und Pilzen in einer Probe durch
a) Inkontaktbringen der Probe mit einem chemisch definierten Zellkulturmedium, das 0,01 bis 10 g/l Glutamin, 0,01 bis 3 g/l Cystein und/oder Cystin, 0,1 bis 300 mg/l Adenin, 0,01 bis 100 mg/l Guanin, 0,01 bis 10 g/l Aminobenzoesäure, Nikotinamid-Adenin-Dinukleotid, und 0,01 bis 100 mg/l eines Eisen(III)salzes umfasst.
b) Inkubieren des Zellkulturmediums aus Schritt a)
c) Nachweisen der Anwesenheit der Prokaryoten, Hefen und Pilze im Zellkulturmedium.

8. Verfahren nach Anspruch 7, **dadurch gekennzeichnet, dass** die Inkubation in Schritt a) für weniger als 30 Stunden durchgeführt wird.

9. Verfahren nach Anspruch 7 oder 8, **dadurch gekennzeichnet, dass** das Vorhandensein der Prokaryonten, Hefen und Pilze über ihr Wachstum oder über eine durch die Prokaryoten, Hefen und Pilze induzierte Änderung der Farbe oder des pH-Werts nachgewiesen wird.

10. Verfahren nach einem oder mehreren der Ansprüche 7 bis 9, **dadurch gekennzeichnet, dass** das Medium ein Geliermittel umfasst.

11. Verwendung ein chemisch definierten Zellkulturmediums, das 0,01 bis 10 g/l Glutamin, 0,01 bis 3 g/l Cystein und/oder Cystin, 0,1 bis 300 mg/l Adenin, 0,01 bis 100 mg/l Guanin, 0,01 bis 10 g/l Aminobenzoesäure, Nikotinamid-Adenin-Dinukleotid, und 0,01 bis 100 mg/l eines Eisen(III)-salzes umfasst, zum Züchten von Prokaryoten, Hefen und Pilzen für das Testen auf Keimbelastung, Sterilität, Umwelt oder Medienabfüllung.

12. Zellkulturmedium-Ergänzung, die 0,01 bis 10 g/l Glutamin, 0,01 bis 3 g/l Cystein und/oder Cystin, 0,1 bis 300 mg/l Adenin, 0,01 bis 100 mg/l Guanin, 0,01 bis 10 g/l Aminobenzoesäure, Nikotinamid-Adenin-Dinukleotid, und 0,01 bis 100 mg/l eines Eisen(III)salzes umfasst.

## Revendications

1. Méthode de culture de procaryotes, de levures et de champignons dans un milieu de culture cellulaire chimiquement défini, **caractérisé en ce que** les cellules sont incubées dans un milieu de culture cellulaire comprenant de 0,01 à 10 g/l de glutamine, de 0,01 à 3 g/l de cystéine et/ou de cystine, de 0,1 à 300 mg/l d'adénine, de 0,01 à 100 mg/l de guanine, de 0,01 à 10 g/l d'acide aminobenzoïque, du nicotinamide adénine dinucléotide et de 0,01 à 100 mg/l d'un sel de fer(III).

2. Méthode selon la revendication 1, **caractérisée en ce que** le milieu de culture cellulaire comprend un ou plusieurs composants saccharidiques, un ou plusieurs acides aminés, un(e) ou plusieurs vitamines ou précurseurs de vitamines, un ou plusieurs sels, un ou plusieurs composants de tampon, un ou plusieurs cofacteurs, et un ou plusieurs composants d'acide nucléique.

3. Méthode selon une ou plusieurs parmi les revendications 1 à 2, **caractérisée en ce que** le milieu de culture cellulaire comprend un agent gélifiant.

4. Méthode selon une ou plusieurs parmi les revendications 1 à 3, **caractérisée en ce que** les procaryotes qui sont cultivés comprennent une ou plusieurs parmi les souches suivantes : *Staphylococcus aureus, Bacillus subtilis, Escherichia coli, Streptococcus pyogenes, Pseudomonas aeruginosa.*

5. Méthode selon une ou plusieurs parmi les revendications 1 à 4, **caractérisée en ce que** les levures et les champignons qui sont cultivés comprennent une ou plusieurs parmi les souches suivantes : *Candida albicans, Aspergillus brasiliensis* et/ou *Saccharomyces cerevisiae.*

6. Méthode selon une ou plusieurs parmi les revendications 1 à 5, **caractérisée en ce que** les procaryotes, les levures et les champignons comprennent au moins une souche fastidieuse.

7. Méthode de détection de procaryotes, de levures et de champignons dans un échantillon, par
a) la mise en contact de l'échantillon avec un milieu de culture cellulaire chimiquement défini comprenant de 0,01 à 10 g/l de glutamine, de 0,01 à 3 g/l de cystéine et/ou de cystine, de 0,1 à 300 mg/l d'adénine, de 0,01 à 100 mg/l de guanine, de 0,01 à 10 g/l d'acide aminobenzoïque, du nicotinamide adénine dinucléotide et de 0,01 à 100 mg/l d'un sel de fer(III) ;
b) l'incubation du milieu de culture cellulaire de l'étape a) ;
c) la détection de la présence des procaryotes, des levures et des champignons dans le milieu de culture cellulaire.

8. Méthode selon la revendication 7, **caractérisée en ce que** l'incubation dans l'étape b) est mise en œuvre pendant moins de 30 heures.

9. Méthode selon la revendication 7 ou 8, **caractérisée en ce que** la présence des procaryotes, des levures et des champignons est détectée via leur croissance ou via un changement de couleur ou de pH induit par les procaryotes, les levures et les champignons.

10. Méthode selon une ou plusieurs parmi les revendications 7 à 9, **caractérisée en ce que** le milieu comprend un agent gélifiant.

11. Utilisation d'un milieu de culture cellulaire chimiquement défini comprenant de 0,01 à 10 g/l de glutamine, de 0,01 à 3 g/l de cystéine et/ou de cystine, de 0,1 à 300 mg/l d'adénine, de 0,01 à 100 mg/l de guanine, de 0,01 à 10 g/l d'acide aminobenzoïque, du nicotinamide adénine dinucléotide et de 0,01 à 100 mg/l d'un sel de fer(III), pour la culture de procaryotes, de levures et de champignons à des fins de test de charge microbienne, de stérilité, environnemental ou de remplissage aseptique.

12. Complément de milieu de culture cellulaire comprenant de 0,01 à 10 g/l de glutamine, de 0,01 à 3 g/l de cystéine et/ou de cystine, de 0,1 à 300 mg/l d'adénine, de 0,01 à 100 mg/l de guanine, de 0,01 à 10 g/l d'acide aminobenzoïque, du nicotinamide adénine dinucléotide et de 0,01 à 100 mg/l d'un sel de fer(III).
